# EUROPÄISCHE PATENTSCHRIFT

(11) **EP 3 481 276 B1**
(45) Veröffentlichungstag und Bekanntmachung des Hinweises auf die Patenterteilung: **17.06.2020**
(21) Anmeldenummer: 17735494.1
(22) Anmeldetag: 04.07.2017
(51) Int. Cl.: A61B 5/00, A61B 5/103, A61Q 5/00

(54) **VERFAHREN ZUM ERMITTELN EINER NUTZERSPEZIFISCHEN HAARBEHANDLUNG**
METHOD FOR ESTABLISHING A USER-SPECIFIC HAIR CARE TREATMENT
PROCÉDÉ POUR ÉTABLIR UN TRAITEMENT CAPILLAIRE SPÉCIFIQUE POUR UN UTILISATEUR

(30) Priorität: 05.07.2016 DE 102016212202; 11.11.2016 DE 102016222180
(43) Veröffentlichungstag der Anmeldung: 15.05.2019
(73) Patentinhaber: Henkel AG & Co. KGaA, 40589 Düsseldorf (DE)
(72) Erfinder: KNÜBEL, Georg, 40219 Düsseldorf (DE)
(86) Internationale Anmeldenummer: PCT/EP2017/066574
(87) Internationale Veröffentlichungsnummer: WO 2018/007354

(56) Entgegenhaltungen:
- WO-A1-2015/166403
- JP-A- 2010 112 788
- US-A1- 2002 010 556
- US-A1- 2006 281 994
- US-A1- 2014 118 521

## Beschreibung

Die Erfindung betrifft ein Verfahren zum Ermitteln einer nutzerspezifischen Haarbehandlung.

Bei einer Behandlung von Haar mit kosmetischen Produkten kann eine Wirkung des Produkts, z.B. eine Intensität einer Färbung, stark vom Schädigungsgrad des Haars abhängen. Zudem ist geschädigtes Haar oftmals schwer handhabbar und glanzlos.

Es gibt viele verschiede Haarpflegeprodukte am Markt, welche unterschiedlich Haareigenschaften oder -parameter, wie beispielsweise den Glanz, verbessern sollen. In vielen Fällen weiß der Anwender solcher Produkte aber nicht, wie stark und auf weiche Weise seine Haare geschädigt sind. Dies kann dazu führen, dass der Anwender zu in seinem speziellen Fall weniger geeigneten Produkten greift und nach ihrer Anwendung mit deren Wirksamkeit unzufrieden ist.

Deshalb kann eine Ermittlung einer Schädigung des Haars von großer Bedeutung sein.

Haar kann durch natürliche oder künstlich herbeigeführte Vorgänge geschädigt werden. Der wichtigste Schädigungstyp kann dabei eine oxidative Schädigung sein.

Die natürlichen Vorgänge können beispielsweise eine kombinierte (z.B. gleichzeitige) Einwirkung von UV-Licht und Sauerstoff (O₂) auf das Haar aufweisen.

Die künstlich herbeigeführten Vorgänge können dabei beispielsweise ein Anwenden von Haarfärbemitteln (auch als Colorationen bezeichnet), ein Blondieren, und/oder ein Erzeugen einer Dauerwelle umfassen.

Dabei kann neben erwünschten kosmetischen Effekten, wie z.B. einer Aufhellung des Haars, auch eine starke Schädigung des Haars auftreten, beispielsweise bei einer Verwendung von Oxidationsmitteln.

Der Schädigungsvorgang kann dabei ursächlich durch eine Oxidation von Aminosäuren, beispielsweise eine Oxidation der im Haar vorkommenden Aminosäuren Tryptophan, Methionin, Tyrosin, Histidin und Lysin erfolgen.

Die Oxidation dieser Aminosäuren kann die mechanische Stabilität des Haars zerstören und bei mehrfachen Anwendungen sogar zu einem vollständigen Haarbruch führen. Allerdings können bereits vorher makroskopisch wahrnehmbare, z.B. fühlbare, Eigenschaften des Haars, beispielsweise eine Oberflächenrauigkeit, negativ beeinflusst werden. Auch Ergebnisse kosmetischer Behandlungen, insbesondere schädigender Verfahren, können bereits in einem frühen Schädigungsstadium massiv verändert sein im Vergleich zu einem Ergebnis bei ungeschädigtem Haar. Außerdem kann es zu einer Vergilbung bei grauem Haar kommen.
Da die Oxidationsprodukte der genannten Aminosäuren bekannt sind, ist es auch möglich deren Gehalt und somit den Schädigungsgrad von Haaren exakt zu ermitteln.
Im akademischen und industriellen Bereich stehen einem Forscher oder Entwickler eine Vielzahl von physikalischen und chemisch-analytischen Verfahren zur Verfügung, um eine Ermittlung des Schädigungsgrads durchzuführen, beispielsweise eine quantitative Ermittlung des oxidativen Schädigungsgrads.
Herkömmlich werden hierbei chromatographische Verfahren verwendet, wie beispielsweise Hochleistungsflüssigkeitschromatographie (HPLC), nach einem aufwändigen sauren, basischen oder enzymatischen hydrolytischen Aufschluss der Haarprobe. Alternativ können colorimetrische Verfahren verwendet werden.
Allerdings sind all diese Verfahren kompliziert und apparativ aufwändig, so dass sie einem Endverbraucher nicht zur Verfügung stehen.
Die Dokumente US2006/281994 A1 und US 2002/010556 A1 offenbaren Verfahren zur Bestimmung der Haarschädigung. Das Dokument WO 2015/166403 A1 beschreibt ein Verfahren zur Bestimmung der Zusammensetzung eines Haarfärbemittels. Das Dokument JP2010112788 A offenbart ein verfahren zur Bestimmung der Haargualität.
Immer mehr Anwender von Produkten wünschen sich ein auf ihre individuellen Bedürfnisse abgestimmtes Produkt. Dies gilt insbesondere auch für Schönheitsprodukte wie Haut- und/oder Haarbehandlungsmittel.
Es war deshalb eine Aufgabe der vorliegenden Erfindung ein Verfahren zur Ermittlung individueller Haarbehandlungsanweisungen bereitzustellen, welches einem Endverbraucher auf einfache Weise erlaubt, den Gehalt an Oxidationsprodukt einer Aminosäure in seinem Haar zu bestimmen und eine darauf abgestimmte Haarbehandlungsanweisung zu erhalten.
Diese Aufgabe wird gelöst durch ein Verfahren zur Ermittlung einer individualisierten Haarbehandlung mit den Schritten:
a) Ermitteln eines Gehalts an Oxidationsprodukt einer Aminosäure mehrerer Proben unterschiedlich geschädigter Keratinfasern mittels eines chromatographischen oder colorimetrischen Verfahrens;
b) Aufnahme von Nahinfrarot- und/oder Infrarotspektren der Proben unterschiedlich geschädigter Keratinfasern;
c) Erstellen eines Kalibriermodells, das eine Korrelation zwischen Nahinfrarot- und/oder Infrarotspektren und dem Gehalt an Oxidationsprodukt einer Aminosäure herstellt;
d) Aufnahme von Nahinfrarot- und/oder Infrarotspektren der Keratinfasern eines Individuums;
e) Bestimmung eines Schädigungsgrades der Keratinfasern dieses Individuums anhand des Kalibriermodells;
f) Ausgabe individueller Behandlungsanweisungen zu den Keratinfasern des Individuums in Abhängigkeit vom ermittelten Schädigungsgrad,
wobei die Aminosäure ausgewählt ist aus der Gruppe bestehend aus Tryptophan, Methionin, Tyrosin, Histidin und Lysin.

Die Begriffe "Keratinfaser" und "Haar" umfassen im Rahmen dieser Anmeldung Pelze, Wolle und Federn, insbesondere aber menschliche Haare. Die Begriffe "Haar" und "Keratinfaser" werden synonym verwendet.

Ein "Nutzer" oder "Anwender" kann im Rahmen dieser Anmeldung das Individuum sein, dessen Keratinfasern untersucht werden und für die eine individualisierte Haarbehandlung ermittelt wird. Alternativ kann ein "Nutzer" oder "Anwender" ein Dritter sein, der die Keratinfasern eines Individuums untersucht und für diese eine individualisierte Haarbehandlung ermittelt.

Die Nahinfrarot- und/oder Infrarotspektroskopie erlaubt die direkte, zerstörungsfreie Bestimmung des Gehalts an Oxidationsprodukt einer Aminosäure ohne eine aufwendige Probenvorbereitung und ohne das Haar durch die Analyse in seiner Struktur zu verändern oder zu zerstören. Dies hat den Vorteil, dass das Ergebnis sehr schnell erhalten wird und die Haare gleich nach der Bestimmung des Gehalts an Oxidationsprodukt einer Behandlung unterzogen werden können. Ein weiterer Vorteil ist, dass die Nahinfrarot- und/oder Infrarotspektroskopie an Keratinfasern, welche sich am Kopf des Individuums befinden, ausgeführt werden kann.

Direkte Oxidationsprodukte von Tryptophan umfassen 5-Hydroxytryptophan und Oxindolylalanin (2-Hydroxytryptophan). Weitere Oxidationsprodukte entstehen durch Spaltung des Indol-Rings: N-Formylkynurenin, Kynurenin und Hydroxykynurenin. Noch weitere Oxidationsprodukte entstehen durch erneute Cyclisierungen: Carboline und Hexahydropyrroloindol.

In einer bevorzugten Ausführungsform der Erfindung ist Oxidationsprodukt einer Aminosäure ausgewählt aus der Gruppe bestehend aus 5-Hydroxytryptophan, Oxindolylalanin, N-Formylkynurenin, Kynurenin, Hydroxykynurenin, Carboline und Hexahydropyrroloindol.

Die Oxidationsprodukte von Methionin umfassen Methioninsulfoxid (2-Amino-4-(methylsulfinyl)butansäure) und/oder Methioninsulfon.

In einer weiteren bevorzugten Ausführungsform der Erfindung ist das Oxidationsprodukt einer Aminosäure ausgewählt aus der Gruppe bestehend aus Methioninsulfoxid und Methioninsulfon. Das beanspruchte Verfahren verlangt zunächst die Erstellung eines Kalibriermodells. Dazu wird in einem Schritt a) zunächst der Gehalt an Oxidationsprodukt einer Aminosäure mehrerer Proben unterschiedlich geschädigter Keratinfasern mittels eines chromatographischen, beispielsweise HPLC, oder eines colorimetrischen Verfahrens bestimmt.

In einem Schritt b) werden dann Nahinfrarot- und/oder Infrarotspektren der Proben unterschiedlich geschädigter Keratinfasern aufgenommen.

Die Nahinfrarot- (NIR-) und/oder ein Infrarot-(IR-)Spektren können beispielsweise mittels ATR-(Nah-)Infrarotspektroskopie (von Englisch "attenuated total reflection", auf Deutsch "abgeschwächte Totalreflexion") gewonnen werden.

In Schritt c) wird ein Kalibriermodell erstellt, das eine Korrelation zwischen den Nahinfrarot- und/oder Infrarotspektren der Proben (Kalibrierspektren) und dem Gehalt an Oxidationsprodukt einer Aminosäure der Proben, der mittels eines unabhängigen chromatographischen oder colorimetrischen Verfahrens ermittelt wurde, herstellt. Das Erstellen des Kalibriermodells kann auch umfassen, dass für die Mehrzahl von Kalibrierhaarproben ein Kalibrierspektrum von zumindest einem Teil des Nahinfrarot- und/oder Infrarotlichts, welches während eines Belichtens der Kalibrierhaarprobe mit Nahinfrarot- und/oder Infrarotlicht von der Kalibrierhaarprobe reflektiert und/oder gestreut wird, aufgenommen wird, ein Schädigungsgrad der Kalibrierhaarprobe mittels eines unabhängigen chromatographischen oder colorimetrischen Verfahrens ermittelt wird, ein Schädigungsgrads zum Kalibrierspektrum zugewiesen wird und eine Korrelation zwischen der Mehrzahl von Kalibrierspektren und der Mehrzahl von Schädigungsgraden ermittelt wird.

Die Schritte a) und b) müssen nicht zwingend nacheinander und in dieser Reihenfolge durchgeführt werden. So kann zuerst Schritt b) und dann Schritt a) durchgeführt werden.

In einer bevorzugten Ausführungsform liegt das Kalibriermodell aus den Schritten a) bis c) als gespeicherte Information auf einem lokalen Datenträger oder in einer Cloud vor. Ein lokaler Datenträger im Sinne dieser Anmeldung umfasst alle physischen Trägersubstanzen, auf denen Daten festgehalten werden können. In einer besonders bevorzugten Ausführungsform ist der Datenträger identisch mit der Datenverarbeitungsvorrichtung an die das (N)IR-Spektrometer zur Aufnahme von Nahinfrarot- und/oder Infrarotspektren der Keratinfasern angeschlossen ist. Dies kann insbesondere ein Smartphone, ein Tablet, ein Laptop oder ein Computer sein. In einer alternativen Ausführungsform liegt das Kalibriermodell aus den Schritten a) bis c) als gespeicherte Information in einer Cloud vor.

Durch eine Anwendung von mathematischen Modellen kann mittels Vermessung von KalibrierHaarproben, welche einen anhand eines bekannten analytischen Verfahrens ermittelten Gehalt an Oxidationsprodukt einer Aminosäure aufweisen, ein mathematisches Modell erstellt werden, welches dann bei einer Haarprobe, auch als Tresse bezeichnet, des Verbrauchers anhand des aufgenommenen NIR- bzw. IR-Spektrums eine Berechnung eines Gehalts an Oxidationsprodukt einer Aminosäure, und damit der Haarschädigung, erlaubt. Eine Analyse des Spektrums und eine Anwendung des Modells kann dabei beispielsweise (mit geeigneten Apps) mittels bekannter Datenverarbeitungsvorrichtungen wie Smartphones, Tablet o.ä. ausgeführt werden.

Das mathematische Modell kann ein künstliches System sein, das beispielsweise aus den KalibrierHaarproben lernt und diese nach Beendigung der Lernphase verallgemeinern kann. Das heißt, es werden nicht einfach die Beispiele auswendig gelernt, sondern es werden Muster und Gesetzmäßigkeiten in den Lerndaten erkannt. Hierzu können unterschiedliche Ansätze verfolgt werden. Beispielsweise kann ein überwachtes Lernen, ein teilüberwachtes Lernen, ein unüberwachtes Lernen, ein bestärktes Lernen und/oder ein aktives Lernen eingesetzt werden insbesondere in Verbindung mit *deep learning*-Verfahren. Ein überwachtes Lernen kann beispielsweise mittels eines künstlichen neuronalen Netzes (etwa einem rekurrenten neuronalen Netz) oder mittels einer Support Vector Machine erfolgen. Auch ein unüberwachtes Lernen kann beispielsweise mittels eines künstlichen neuronales Netzes (beispielsweis eines Autoencoders) erfolgen.

Zur Optimierung des Kalibriermodells können weitere Faktoren, insbesondere kategoriale Faktoren, wie beispielsweise Ethnizität des Individuums, Alter des Individuums (als Kategorie oder metrisch), Haarfarbe des Individuums (als Kategorie oder metrisch), berücksichtigt werden.

Im weiteren Verfahren wird in Schritt d) ein Nahinfrarot- und/oder Infrarotspektren der Keratinfasern eines Individuums aufgenommen. Dies kann beispielsweise derart erfolgen, dass während eines Belichtens einer Haarprobe des Haars des Individuums mit Nahinfrarot- und/oder Infrarotlicht ein Spektrum von zumindest einem Teil des Nahinfrarot- und/oder Infrarotlichts, welches von der Haarprobe reflektiert und/oder gestreut wird aufgenommen wird. Vorzugsweise werden in Schritt d) mehrere Nahinfrarot- und/oder Infrarotspektren pro Messpunkt aufgenommen und jeweils gemittelt.

In Schritt e) wird der Schädigungsgrad der Keratinfasern des Individuums anhand des in den Schritten a) bis c) erstellten Kalibriermodells bestimmt.

Dazu wird beispielsweise zumindest ein Teil des Nahinfrarot- und/oder Infrarotspektrums vom Haar des Inviduums mit dem Kalibriermodell verglichen und ein Schädigungsgrad des Haars ermittelt/zugewiesen.

In einer weiteren vorteilhaften Ausführungsform erfolgt die Aufnahme der Nahinfrarot- und/oder Infrarotspektren der Keratinfasern eines Individuums an unterschiedlichen Positionen entlang der Keratinfasern. So kann die Aufnahme der Nahinfrarot- und/oder Infrarotspektren am Ansatz und/oder in der Mitte und/oder an den Spitzen der Keratinfasern erfolgen. Entsprechend kann für jede dieser Positionen ein eigener Schädigungsgrad ermittelt werden.

Die Nahinfrarotspektroskopie basiert wie andere Schwingungsspektroskopien auf der Anregung von Molekülschwingungen durch elektromagnetische Strahlung im (nahen) Infrarotbereich. Bei der Nahinfrarotspektroskopie findet die Detektion im nahen Infrarot (760-2500 nm oder ca. 13.000-4.000 cm⁻¹) statt. Im Folgenden wird für Licht mit einer Wellenzahl in einem Bereich von 12500 bis 4000 cm⁻¹ der Begriff Nahinfrarot (NIR) verwendet, und für Licht mit einer Wellenzahl in einem Bereich von 3999 bis 400 cm⁻¹ der Begriff Infrarot (IR).

Durch Verwendung miniaturisierter (N)IR-Spektrometer und/oder (N)IR-Sensoren sowie deren Anschluss an eine mobile Datenverarbeitungsvorrichtung kann das Verfahren und insbesondere Schritt d) des Verfahrens beispielsweise im privaten Bereich durch das Individuum selber, durch eine beliebige Person an einem Verkaufspunkt ("point of sale", POS) von Haarbehandlungsmitteln oder durch einen Friseur durchgeführt werden. In einer bevorzugten Ausführungsform ist die mobile Datenverarbeitungsvorrichtung ein smartes Endgerät, zum Beispiel ein Smartphone, ein Tablet oder ein Laptop.

Die (N)IR-Spektrometer können insbesondere auch in mobiler Form bereitgestellt werden, beispielsweise in Form von Hand-Spektrometern ("handheld") oder Aufsatz-Spektrometern.

Ein Beispiel für ein geeignetes Hand-Spektrometer ist das "MicroNIR OnSite" der Firma Viavi Solutions Inc. Dieses Spektrometer wird von einem Tablet oder einem Laptop über einen USB-Anschluss mit Strom versorgt und gesteuert. Dieses Hand-Spektrometer ermöglicht mit einer Messzeit von zwischen 0,25 und 0,5 Sekunden die Aufnahme der Nahinfrarot- und/oder Infrarotspektren der Keratinfasern eines Individuums in Echtzeit. Das Spektrometer weist zwei integrierte Vakuum-Wolfram-Lampen und ein InGaAs-Photodioden-Array mit 128 Pixeln auf. Das "MicroNIR OnSite" arbeitet in einem Wellenzahlenbereich von 6060 bis 10526 cm⁻¹. Der Abstand zwischen den Keratinfasern und dem Glas des Handspektrometers kann zwischen 0 und 15 mm, wobei ein Abstand von 3 mm bevorzugt ist.

In einer Ausführungsform der Erfindung wird das ganze Verfahren zur Ermittlung einer individualisierten Haarbehandlung durch das Tablet oder den Laptop durchgeführt, welcher das "MicroNIR OnSite"-Spektrometer mit Strom versorgt und steuert. Alternativ können die gewonnenen spektroskopischen Daten an eine weitere (mobile) Datenverarbeitungsvorrichtung, insbesondere ein weiteres smartes Endgerät, gesendet werden, welches dann das Verfahren zur Ermittlung einer individualisierten Haarbehandlung durchführt. Die Übertragung der spektroskopischen Daten kann beispielsweise kabellos mittels WLAN (WiFi) oder Bluetooth erfolgen.

Ein weiteres geeignetes Hand-Spektrometer ist das "i-Spec Nano" der Firma B&W Tek. Das Spektrometer wird über einen USB-Anschluss und einer daran angeschlossenen (mobilen) Datenverarbeitungsvorrichtung oder über eine Batterie mit Strom versorgt. Das Spektrometer weist eine Lichtquelle auf und arbeitet in einem Wellenzahlenbereich von 4545 bis 7692 cm⁻¹. Die Übertragung der spektroskopischen Daten an eine (mobile) Datenverarbeitungsvorrichtung, welche dann das Verfahren zur Ermittlung einer individualisierten Haarbehandlung durchführt, kann kabellos mittels WLAN (WiFi) oder Bluetooth erfolgen.

Ebenfalls geeignet ist das Hand-Spekrometer "QualitySpec Trek" der Firma ASD Inc. Dieses arbeitet in einem Wellenzahlenbereich von 28571 bis 400 cm⁻¹.

Ein weiteres geeignetes Hand-Spektrometer ist das "SCiO by Consumer Physics", welches mit Hilfe der integrierten App "SpectroScan" die spektroskopischen Daten auf einem smarten Endgerät anzeigt. Das Hand-Spektrometer arbeitet im kurzwelligen Bereich des NIR und zwar bei Wellenzahlen von 9090 bis 14285 cm⁻¹ (entspricht 700 bis 1100 nm). Die Auswertung der gemessenen Daten erfolgt mit Hilfe einer Cloud, in der beispielsweise eine Materialdatenbank, chemometrische Modelle und Algorithmen gespeichert sind.

Noch weitere geeignete Hand-Spektrometer sind von der Firma Attonics Systems erhältlich, welche entweder in Wellenzahlenbereichen von 9090 bis 26.315 cm⁻¹ (VIS-NIR) oder von 3333 bis 10.000 cm⁻¹ (NIR) arbeiten. Diese Spektrometer basieren auf Interferometern und weisen einen hohen Lichtdurchsatz und eine hohe spektrale Auflösung (< 5 nm für VIS-NIR-Spektrometer und < 20 nm für das NIR-Spektrometer) auf. Die Spektrometer weisen einen Multi-Phase Shift Array (MPA) Chip und eine optische Anordnung in einem kreisförmigen Rohr auf. Ferner sind die Spektrometer kompatibel mit mobilen Datenverarbeitungsvorrichtungen.

Weitere Beispiele für VIS-NIR-Spektrometer sind die Miniaturspektrometer "USB2000-VIS-NIR" und "USB4000-VIS-NIR" der Firma Ocean Optics. Dieses Spektrometer arbeitet mit einem Wellenlängenbereich von 350 bis 1000 nm. Die Spektrometer werden über einen USB-Anschluss mit einer Datenverarbeitungsvorrichtung verbunden werden.

Daneben existieren eine Reihe von NIR-Sensoren oder NIR-Auswertungsmodulen, welche in Hand-Spektrometer verwendet werden können. Geeignete NIR-Auswertungsmodule sind die Module "DLP® NIRscan" und "DLP® NIRscan Nano" der Firma Texas Instruments. Diese weisen zwei Wolfram-Lampen und InGaAs-Photodioden als Detektoren auf. Das Modul "DLP® NIRscan" arbeitet im Wellenzahlenbereich von 4016 bis 7407 cm⁻¹ und das Modul "DLP® NIRscan Nano" im Bereich von 5882 bis 11111 cm⁻¹. Die Kommunikation der spektroskopischen Daten erfolgt kabellos über Bluetooth Low Energy. Mit Hilfe von "Software Developer Kits" (SDK), beispielsweise das Open Source SDK von KST Technologies, können Apps entwickelt werden, die die spektroskopischen Daten auswerten oder weiter verarbeiten.

Weitere geeignete NIR-Sensoren sind unter der Bezeichnung "NeoSpectra" von Si-Ware Systems erhältlich. Konkrete Sensoren umfassen: NeoSpectra SW62221-1.7, NeoSpectra SW62221-2.1 und NeoSpectra SW62221-2.5, welche in unterschiedlichen Wellenzahlenbereichen arbeiten.

In einer bevorzugten Ausführungsform wird Schritt d) durch eine mobile Datenverarbeitungsvorrichtung, insbesondere ein smartes Endgerät, gesteuert. Dabei ist es bevorzugt, dass das Verfahren eine auf dem smarten Endgerät vorab installierte App gesteuert wird. Smarte Endgeräte umfassen insbesondere Smartphones oder Tablets.

Unter einer "App" wird im Rahmen dieser Anmeldung ein Computerprogramm bezeichnet, das genutzt wird, um eine nicht systemtechnische Funktionalität zu bearbeiten oder zu unterstützen. Der Begriff "App" umfasst insbesondere Anwendungssoftware für smarte Endgeräte wie Smartphones und Tablets ("mobile App") als auch Desktop-Anwendungssoftware. Die App kann eine native App sein, die nur auf einer Plattform funktioniert, oder eine plattformunabhängige Web-, Hybrid- oder Cross-Plattform-App.

Die App kann besonders bevorzugt über einen QR-Code, einen NFC-Chip, einen Barcode oder einen RFID-Chip direkt bei einem Friseur oder an einem Verkaufspunkt ("point of sale", POS) von Haarbehandlungsmitteln heruntergeladen werden.

Alternativ kann die App, insbesondere im Fall, dass sie auf einem smarten Endgerät installiert werden soll, über eine in das jeweilige Betriebssystem des smarten Endgeräts integrierte Internet-Vertriebsplattform heruntergeladen werden. Im Fall eines smarten Endgeräts mit dem Betriebssystem "Apple iOS" kann dies beispielsweise der "App Store" sein oder im Fall eines smarten Endgeräts mit dem Betriebssystem "Android" kann dies der "Google Play Store" sein.

In einer Ausführungsform enthält der QR-Code, der NFC-Chip, der Barcode oder der RFID-Chip einen Weblink, welcher den Anwender des Verfahrens auf eine Internetseite führt, von der der Anwender des Verfahrens die App herunterladen kann.

Es ist ferner bevorzugt, wenn der in dem Verfahren ermittelte Schädigungsgrad in "% geschädigt" oder in einer relativen Angabe ("sehr stark, stark, mittel, schwach und gar nicht") ausgegeben wird. Eine Ausgabe in "% geschädigt" ermöglicht einen direkten Vergleich der zu unterschiedlichen Zeitpunkten jeweils für ein Individuum ermittelten Schädigungsgrade. So kann beispielsweise auch die Wirksamkeit der Behandlung zeitlich verfolgt werden.

Die Ausgabe des Schädigungsgrads kann optisch, beispielsweise mittels einer Anzeigevorrichtung der (mobilen) Datenverarbeitungsvorrichtung, oder akustisch, beispielsweise mittels einer Sprachansage über einen Lautsprecher, erfolgen.

In einer besonders bevorzugten Ausführungsform des Verfahrens umfasst die individuelle Behandlungsanweisung die Empfehlung von Blondiermitteln und/oder Haarfärbemitteln und/oder Haarpflegeprodukten und/oder Haarstylingmitteln.

Die Empfehlung kann dabei die Anzeige oder Ansage eines konkreten Produktnamens eines Blondiermittels und/oder Haarfärbemittels und/oder Haarpflegeprodukts und/oder Haarstylingmittels umfassen. Alternativ kann die Empfehlung die Anzeige oder Ansage einer Produktlinie oder -serie, insbesondere eine Blondiermittellinie/-serie und/oder Haarfärbemittellinie/-serie und/oder Haarpflegeproduktlinie/-serie und/oder Haarstylingmittellinie/-serie, eines Herstellers umfassen.

In einer alternativen Ausführungsform des Verfahrens umfasst die individuelle Behandlungsanweisung die Empfehlung, für einen bestimmten Zeitraum von Blondierungen und/oder oxidativem Färben und/oder permanenten Verformungen und/oder Hitzebehandlungen Abstand zu nehmen. Diese individuelle Behandlungsanweisung kann insbesondere bei Überschreiten einer bestimmten Prozentzahl für den ermittelten Schädigungsgrad erfolgen oder bei der relativen Einstufung des Schädigungsgrads in den Bereichen "sehr stark" und/oder "stark".

Unter den Begriff "permanente Verformung" fallen alle Verfahren zur Lockung von glattem Haar oder Glättung von gelocktem Haar. Dieses können Dauerwell-Verfahren oder chemische Glättungsverfahren sein. Neben dem Einsatz von Chemikalien, können auch Hitzebehandlungen Keratinfasern weiter schädigen. Entsprechend kann die individuelle Behandlungsanweisung die Empfehlung umfassen, von Hitzebehandlungen wie beispielsweise dem Einsatz von Lockenstäben oder Glätteisen, für einen bestimmten Zeitraum abzusehen.

Weisen die Keratinfasern beispielsweise einen leichten bis mittleren Schädigungsgrad auf, kann in einer weiteren, bevorzugten Ausführungsform des Verfahrens die individuelle Behandlungsanweisung die Empfehlung umfassen, für einen bestimmten Zeitraum die Haarfarbe nur um eine maximale Zahl von Nuancen aufzuhellen und/oder oxidativ zu färben.

In einer weiteren Ausführungsform der Erfindung kann die individuelle Behandlungsanweisung eine Dosierungsempfehlung für eine Blondierung und/oder eine oxidative Färbung und/oder die Vorhersage eines Resultats einer Blondierung und/oder oxidativen Färbung umfassen.

Bei einer Blondierung oder beim oxidativen Färben von Keratinfasern wird in einem alkalischen Milieu die Schuppenschicht der Keratinfasern geöffnet und ein - je nach gewünschtem Blondierungs- oder Aufhellungsergebnis - unterschiedlich hoch dosierter Wasserstoffperoxid-Cocktail löst die natürlichen (Farb-)Pigmente mehr oder weniger stark auf. Die Keratinfaser wird umso heller, je höher dosiert das Wasserstoffperoxid ist. Aufgrund dieser Wirkungsweise schädigen Blondierungen oder oxidative Färbungen Keratinfasern immer spürbar. Je höher die Konzentration des Wasserstoffperoxids und je länger die Einwirkzeit ist, desto stärker ist die Schädigung.

Bei vorgeschädigten Keratinfasern ist es deshalb vorteilhaft, für einen bestimmten Zeitraum die Haarfarbe nur um eine maximale Zahl von Nuancen aufzuhellen und/oder oxidativ zu färben. Ebenso ist es bei vorgeschädigten Keratinfasern vorteilhaft in einer Dosierungsempfehlung, die Konzentration des Wasserstoffperoxids zu begrenzen, um weitere (starke) Schädigungen durch den Blondierungs- oder Färbevorgang zu vermeiden/reduzieren.

Immer mehr Anwender wünschen bevor sie eine Blondierung oder (oxidative) Färbung vornehmen einen realistischen Eindruck, wie ihre Haare nach einer Blondierung oder (oxidativen) Färbung aussehen. Viele Anbieter von Blondierungen oder (oxidativen) Färbungen bieten deshalb Farbberatungs-Apps an. Mit der App "Schwarzkopf Frisuren Styleguide" lassen sich beispielsweise Haarfarben in Echtzeit vorab testen. Dazu lädt der Anwender die App auf ein smartes Endgerät und nimmt mit der Frontkamera ein Foto seines Kopfes auf. Die Software der App erkennt Gesicht und Kopfform. Der Anwender wählt dann ein konkretes Blondierungsprodukt oder (oxidatives) Färbprodukt aus und bekommt auf der Anzeigevorrichtung des smarten Endgeräts angezeigt, wie er/sie nach Anwendung dieser Blondierung oder (oxidativen) Färbung aussieht.

Zur Vorhersage des Blondierungs- oder Färberesultat wird zunächst die Ausgangshaarfarbe bestimmt und dann basierend auf dem vom Anwender gewählten konkreten Blondierungs- oder Färbeprodukt das Blondierungs- oder Färbungsresultat berechnet. Da der Schädigungsgrad von Haaren einen Einfluss auf das Blondierungs- oder Färberesultat nimmt, liefert das Verfahren zur Ermittlung einer individualisierten Haarbehandlung bessere, insbesondere realistischere, Vorhersagen eines Resultats einer Blondierung und/oder (oxidativen) Färbung. Vorzugsweise ist das Verfahren zum Ermitteln einer individuellen Haarbehandlung Bestandteil einer Farbberatungs-App.

Die individuelle Behandlungsanweisung kann auch darin bestehen, die chemische Zusammensetzung eines Haarbehandlungsmittels, insbesondere eines Blondiermittels, eines Haarfärbemittels, eines Haarpflegeprodukts und/oder Haarstylingmittels zu ermitteln.

In einer weiteren, vorteilhaften Ausführungsform des Verfahren besteht die individuelle Behandlungsanweisung darin, dem Individuum zum/vom Einsatz von Haarbehandlungsprodukten, die der Anwender des Verfahrens und/oder das Individuum anhand von QR-Codes, NFC-Chips, Barcodes oder RFID-Chips identifiziert, zu- oder abzuraten.

In dieser Ausführungsform des Verfahrens kann der Anwender des Verfahrens, beispielsweise ein Friseur oder eine beliebige Person am Verkaufspunkt von Haarbehandlungsmitteln, nach Ermittlung des Schädigungsgrad geeignete oder nicht geeignete Haarbehandlungsmittel über QR-Codes, NFC-Chips, Barcodes oder RFID-Chips, welche beispielsweise am Haarbehandlungsmittel selber oder am Ort dessen Aufbewahrung, beispielsweise am Regal im Friseurgeschäft oder im Verkaufspunkt von Haarbehandlungsmitteln, angebracht sind, ermitteln.

QR-Codes, NFC-Chips, Barcodes oder RFID-Chips ermöglichen, Informationen kabellos zu übertragen.

Als Barcode wird eine optoelektronisch lesbare Schrift bezeichnet, die aus verschieden breiten, parallelen Strichen und Lücken besteht. Die Daten in einem Strichcode werden mit optischen Lesegeräten, wie zum Beispiel Barcodelesegeräten (Scanner) oder Kameras, maschinell eingelesen und elektronisch weiterverarbeitet. Viele smarte Endgeräte weisen eine Software auf, die es ermöglicht, mit der Digitalkamera des smarten Endgeräts zu erfassen und dem Anwender die Code-Information sofort in dekodierter Form anzuzeigen.

Ein QR-Code ("Quick Response") ist ein zweidimensionaler Code, der aus einer quadratischen Matrix aus schwarzen und weißen Quadraten besteht, die die kodierten Daten binär darstellen. Üblicherweise verfügen smarte Endgeräte über eine eingebaute Kamera. Nach dem Fotografieren des QR-Codes wird mit Hilfe einer Software der QR-Code ausgelesen/interpretiert.

NFC-Chips und RFID-Chips sind Sender-Empfänger-Systeme. In diesem Fall muss mindestens ein Kommunikationspartner aktiv sein, also die Kommunikation anregen. Der andere Partner kann ein beispielsweise ein Chip ohne Energieversorgung sein. Dieser passive Teil wird auch Transponder (=Transmitter&Responder) genannt. Neben der aktiv-passiv Kommunikation zwischen beispielsweise einem smarten Endgerät als aktivem Kommunikationspartner und einem Transponder/Chip ist ebenfalls eine aktiv-aktiv Kommunikation möglich.

Die Kopplung/Anregung geschieht durch vom aktiven Kommunikationspartner erzeugte magnetische Wechselfelder mit geringer Reichweite oder durch hochfrequente Radiowellen. Damit werden nicht nur Daten übertragen, sondern auch der Transponder mit Energie versorgt. Der aktive Kommunikationspartner, beispielsweise ein smartes Endgerät, enthält eine Software, die den eigentlichen Leseprozess steuert, und eine sogenannte Middleware mit Schnittstellen zu weiteren (mobilen) Datenverarbeitungsvorrichtungen und/oder Datenbanken.

RFID ("radio-frequency identification") funktioniert über Radiowellen. Die RFID-Technik umfasst ein sehr breites Angebot an verschiedenen Chips und Lesegeräten, welche sich im Wesentlichen durch Speicherkapazität, Herstellverfahren, Kosten, Frequenzbereich und durch die Reichweite unterscheiden.

NFC ("Near Field Communication") ist eine genormte Spezialisierung der RFID-Technik, die speziell für eine Datenübertragung über kurze Distanzen (max. 10 cm) entwickelt wurde.

QR-Codes, NFC-Chips, Barcodes oder RFID-Chips können beispielsweise Informationen enthalten, für welche Schädigungsgrade das zugehörige Haarbehandlungsmittel geeignet oder nicht geeignet ist.

In einer alternativen Ausführungsform des Verfahrens besteht die individuelle Behandlungsanweisung darin, dem Individuum zum Einsatz von Blondiermitteln und/oder Färbemitteln und/oder Pflegeprodukten, die für das Individuum individuell hergestellt werden, zu zuraten und einen Bestellvorgang, vorzugsweise durch Aufrufen einer Internetseite eines Herstellers von individuellen Blondiermitteln und/oder individuellen Färbemitteln und/oder individuellen Pflegeprodukten, einzuleiten.

Immer Kunden wünschen sich ein individuell auf ihre Bedürfnisse abgestimmtes Produkt. Dabei kann es sich um ein speziell für den Kunden hergestelltes Produkt oder ein sogenanntes "mass customized" Produkt handeln. Bei einem "mass customized" Produkt kann durch Variation von wenigen, aus Kundensicht jedoch entscheidenden Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurator abläuft. Dieser Konfigurator hilft dem Kunden bei der Auswahl der Merkmale/Inhaltsstoffe und weist ihn auf die zulässigen/unzulässigen Merkmalskombinationen hin, wobei letztere dann nicht ausgewählt werden können.

Bei Blondiermitteln, Färbemitteln, Pflegeprodukten und Stylingmitteln für Keratinfasern umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators kann beispielsweise die Auswahl chemisch und/oder physikalisch inkompatibler Inhaltsstoffe oder die Auswahl für den ermittelten Schädigungsgrad ungeeigneter Inhaltsstoffe vermieden werden. Umgekehrt kann die Auswahl für den ermittelten Schädigungsgrad geeigneter Inhaltsstoffe durch den Produktkonfigurator vorgegeben oder vorgeschlagen werden.

Es ist auch bevorzugt, dass die individuelle Behandlungsanweisung gespeichert wird und in nachfolgenden Verfahren für eine langfristige Empfehlung genutzt wird.

In einer weiteren Ausführungsform findet vor der Ausgabe der Behandlungsanweisung/Empfehlung an den Anwender ein Datenabgleich zwischen der (mobilen) Datenverarbeitungsvorrichtung, insbesondere dem smarten Endgerät und Daten, die in einer Cloud hinterlegt sind, statt. Dies können beispielsweise Daten von Anwendern mit gleichen oder ähnlichen Schädigungsgraden, Geschlecht, Alter, Verhaltensweisen sowie gegebenenfalls weiteren gleichen oder ähnlichen Keratinfaser-Parametern und den daraus abgeleiteten Empfehlungen/Maßnahmen sein. Durch Aufnahme von Erfahrungswerten, beispielsweise hinsichtlich eines Behandlungserfolgs, weiterer Anwender kann die Beurteilung einer Eignung einer Behandlungsanweisung/Empfehlung bestätigt oder abgeändert werden. Damit kann ermöglicht sein, dass der Anwender immer eine optimale Empfehlung erhält.

In verschiedenen Ausführungsbeispielen kann die individuelle Behandlungsanweisung die Empfehlung umfassen, einen Friseur aufzusuchen. In verschiedenen Ausführungsbeispielen kann direkt über die Software/App, welche die individuelle Behandlungsanweisung ermittelt, ein Buchungsvorgang eingeleitet werden. Dazu können beispielsweise in der Software/App die Kontaktdaten von Friseuren hinterlegt sein und diese dem Nutzer angezeigt werden. Zusätzlich kann über Filter, wie beispielsweise die Postleitzahl, die Auswahl eingeschränkt werden. In verschiedenen Ausführungsbeispielen kann direkt über die Software/App eine Terminbuchung vorgenommen werden. Alternativ kann die Buchung eines Friseurtermins über eine separate Software/App, wie beispielsweise Treatwell, erfolgen.

In einer vorteilhaften Ausführungsform wird das Verfahren nicht auf Basis des Gehalts eines Oxidationsprodukts einer Aminosäure, sondern auf Basis des Gehalts zweier oder mehr Oxidationsprodukte einer Aminosäure durchgeführt.

In einer besonders bevorzugten Ausführungsform wird zusätzlich zum Schädigungsgrad der Keratinfasern deren Kräuselung bestimmt wird und diese bei der individuellen Behandlungsanweisung berücksichtigt.

In einer ebenfalls bevorzugten Ausführungsform wird zusätzlich zum Schädigungsgrad der Keratinfasern deren Dicke bestimmt wird und diese bei der individuellen Behandlungsanweisung berücksichtigt.

Neben dem Schädigungsgrad der Keratinfasern haben weitere Eigenschaften der Keratinfasern Einfluss auf den Erfolg einer Behandlung der Keratinfasern. So können insbesondere die Kräuselung und/oder die Dicke von Keratinfasern einen Einfluss auf den Erfolg einer Behandlung der Keratinfasern nehmen. Die Kräuselung und/oder die Dicke von Keratinfasern nimmt insbesondere Einfluss auf die Behandlung von Keratinfasern mit Pflegemitteln. Da insbesondere für vorgeschädigte Keratinfasern eine erfolgreiche Behandlung mit Pflegeprodukten wünschenswert ist, ist die Berücksichtigung der Kräuselung und/oder der Dicke der individuellen Behandlungsanweisung besonders vorteilhaft.

Die Werte für die Kräuselung und/oder Dicke von Keratinfasern können beispielsweise mit geeigneten Verfahren durch das smarte Endgerät, auf welchem das Verfahren durchgeführt wird, ermittelt werden.

Die Kräuselung von Haaren kann beispielsweise mit Hilfe von Bildbearbeitungs- und Bildverarbeitungsverfahren bestimmt werden. Dazu fotografiert der Anwender des Verfahrens zumindest einen Teil der Haare des Individuums. Geeignete Bildbearbeitungs- und Bildverarbeitungsprogramme wie "ImageJ" bestimmen mit Hilfe geeigneter Plug-Ins den linearen Anteil im Bild. Glattes Haar führt zu einem hohen Anteil an Linearität, stark gekräuseltes Haar zu einem geringen Anteil an Linearität. Der Kräuselungsgrad kann bevorzugt in "% Kräuselung" angegeben werden.

Das Bildbearbeitungs- und Bildverarbeitungsprogramm zur Bestimmung der Kräuselung kann Bestandteil der App zur Durchführung des Verfahrens zur individualisierten Haarbehandlung sein. Alternativ kann die Bestimmung der Kräuselung mit Hilfe von Bildbearbeitungs- und Bildverarbeitungsverfahren mittels separater Verfahren durchgeführt werden. Vorteilhaft wird das separate Verfahren durch eine App, welche sich auch auf der mobilen Datenverarbeitungsvorrichtung, insbesondere dem smarten Endgerät, welche zur Durchführung des Verfahrens zur individualisierten Haarbehandlung eingesetzt wird, durchgeführt.

Die Bestimmung der Kräuselung kann über weitere separate Verfahren, welche nicht mit dem Verfahren zur Ermittlung einer individualisierten Haarbehandlung in Verbindung stehen, oder auch empirisch erfolgen.

Die Information über die Kräuselung kann über eine geeignete Schnittstelle, beispielsweise ein Eingabefenster, welches sich auf dem smarten Endgerät bei der Durchführung des Verfahrens zur individualisierten Haarbehandlung öffnet, bereitgestellt werden. Das Eingabefenster kann dabei relative Kräuselungsgrade, wie beispielsweise "gar nicht", "schwach", "leicht", "mittel", "stark" und "sehr stark", vorgeben und der Anwender des Verfahrens wählt die subjektiv bestimmte Kräuselung aus. Im Fall einer durch ein separates Verfahren bestimmten, prozentualen Kräuselung, kann ein konkreter Zahlenwert, beispielsweise "20 %", eingegeben werden.

Die Dicke kann beispielsweise mit Hilfe eines Zubehörs für smarte Endgeräte bestimmt werden. Dazu wird beispielsweise ein Mikroskop-Aufsatz über die Linse des smarten Endgeräts geklemmt. Beispiele für solche Mikroskop-Aufsätze für smarte Endgeräte sind das "Nurugo Micro" der Firma Nurugo oder "µPeek" des Herstellers Scrona. Der Anwender des Verfahrens bestimmt vor oder nach der Aufnahme von Nahinfrarot- und/oder Infrarotspektren der Keratinfasern des Individuums (Schritt d) auch die Dicke der Keratinfasern des Individuums. Dazu fotografiert er mit Hilfe eines Mikroskop-Aufsatzes 2 bis 20, vorzugsweise 3 bis 15 und insbesondere bevorzugt 5 bis 10, verschiedene Keratinfasern zusammen mit einer Größenreferenz. Mit einer Auswertungssoftware, welche in der App zur Durchführung des Verfahrens zur individualisierten Haarbehandlung integriert sein kann, wird die durchschnittliche Haardicke des Individuums bestimmt.

Ein weiteres, alternatives Verfahren zur Bestimmung der Haardicke, welches mit geeignetem Zubehör durch ein smartes Endgerät durchgeführt werden kann, beinhaltet die Beugung von Laserlicht.

Die Bestimmung der Haardicke kann über separate Verfahren, welche nicht mit dem Verfahren zur Ermittlung einer individualisierten Haarbehandlung in Verbindung stehen, oder auch empirisch erfolgen.

Die Information über die Haardicke kann über eine geeignete Schnittstelle, beispielsweise ein Eingabefenster, welches sich auf dem smarten Endgerät bei der Durchführung des Verfahrens zur individualisierten Haarbehandlung öffnet, bereitgestellt werden. Das Eingabefenster kann dabei relative Haardicken, wie beispielsweise "dünn", "normal" und "dick", vorgeben und der Anwender des Verfahrens wählt die subjektiv bestimmte Haardicke aus. Im Fall einer durch ein separates Verfahren bestimmten, absoluten Haardicke, kann ein Zahlenwert, beispielsweise 80 µm, eingegeben werden.

Dicke Haare und (sehr) stark gekräuselte Haare benötigen zur optimalen Pflege Haarpflegeprodukte mit einem hohen Anteil an Fett- oder Öl-haltigen Inhaltsstoffen, während für dünne und/oder glatte Haare Haarpflegeprodukte mit einem niedrigen Anteil an Fett- oder Öl-haltigen Inhaltsstoffen vorteilhaft sind.

Der Einsatz von Wasserstoffperoxid in einem stark alkalischen Milieu führt bei Blondierungen und oxidativen Färbungen in den meisten Fällen zu einer (weiteren) Schädigung der so behandelten Keratinfasern. Um eine Schädigung zu vermeiden oder zu reduzieren, enthalten Mittel zur Blondierung und/oder zum oxidativen Färben oftmals selber auch Pflegestoffe.

In einer besonders bevorzugten Ausführungsform des Verfahrens umfasst die individuelle Behandlungsanweisung in Abhängigkeit des ermittelten Schädigungsgrads und in Abhängigkeit der Dicke und/oder der Kräuselung die Empfehlung von Blondiermitteln und/oder Haarfärbemitteln und/oder Haarpflegeprodukten und/oder Haarstylingmitteln mit einem auf den Schädigungsgrad und die Dicke und/oder Kräuselung der Keratinfasern abgestimmten Gehalt an Fett- oder Öl-haltigen Inhaltsstoffen.

Fett- und Öl-haltige Inhaltsstoffe umfassen insbesondere Glycerinmono-, -di- oder -triester mit Fettsäuren, Fettsäuren, Fettalkohole, Fettsäuremono- oder-diester mit Fettalkoholen, Pflanzenöle, mineralische Öle, natürliche Wachse und synthetische Wachse.

Eine weitere wichtige Eigenschaft der Keratinfasern, die Einfluss auf den Erfolg einer Behandlung der Keratinfasern nimmt, ist der Grauanteil. Der Grauanteil von Keratinfasern hat insbesondere eine Auswirkung auf das Ergebnis einer Blondierung oder eines oxidativen Färbeprozesses.

Die Bestimmung des Grauanteils kann über separate Verfahren, welche nicht mit dem Verfahren zur Ermittlung einer individualisierten Haarbehandlung in Verbindung stehen, oder auch empirisch erfolgen.

Die Bestimmung des Grauanteils kann beispielsweise mit Hilfe von Bildbearbeitungs- und Bildverarbeitungsverfahren bestimmt werden. Dazu fotografiert der Anwender des Verfahrens zumindest einen Teil der Keratinfasern des Individuums. Vorzugsweise beinhaltet der fotografierte Teil der Keratinfasern weite Teile des Ansatzes. Geeignete Bildbearbeitungs- und Bildverarbeitungsprogramme bestimmen den Grauanteil im Bild. In einer besonders bevorzugten Ausführungsform ist die Bestimmung des Grauanteils integraler Bestandteil des Verfahrens zur individualisierten Haarbehandlung und wird von der App, welche das Verfahren zur Ermittlung einer individualisierten Haarbehandlung durchführt, durchgeführt.

Die Information über den Grauanteil kann auch über eine geeignete Schnittstelle, beispielsweise ein Eingabefenster, welches sich auf dem smarten Endgerät bei der Durchführung des Verfahrens zur individualisierten Haarbehandlung öffnet, bereitgestellt werden. Das Eingabefenster kann dabei prozentuale Grauanteile, wie beispielsweise "10%", "30%", "50%", "70%", "90%" und "100%" vorgeben und der Anwender des Verfahrens wählt den subjektiv bestimmten Grauanteil aus. Im Fall eines durch ein separates Verfahren bestimmten, prozentualen Grauanteil, kann ein Zahlenwert, beispielsweise "68 %", eingegeben werden.

Graue Haare nehmen die beim oxidativ Färben eingesetzten Oxidationsfarbstoffvorprodukte meist schlechter auf als pigmentreichere Haare. Dies führt je nach Grauanteil der Haare zu unterschiedlichen Färberesultaten.

Bei Individuen mit (sehr) stark geschädigtem Haar und keinem oder geringen Grauanteil kann deshalb die individuelle Behandlungsanweisung darin bestehen, dem Individuum vom Einsatz von Blondiermitteln und/oder Färbemitteln abzuraten und zum Einsatz von Färbungen ohne oxidative Behandlung, beispielsweise Tönungen oder Intensiv-Tönungen zu raten.

## Patentansprüche

1. Verfahren zur Ermittlung einer individualisierten Haarbehandlung, **gekennzeichnet durch** die Schritte
a) Ermitteln eines Gehalts an Oxidationsprodukt einer Aminosäure mehrerer Proben unterschiedlich geschädigter Keratinfasern mittels eines chromatographischen oder colorimetrischen Verfahrens;
b) Aufnahme von Nahinfrarot- und/oder Infrarotspektren der Proben unterschiedlich geschädigten Keratinfasern;
c) Erstellen eines Kalibriermodells, das eine Korrelation zwischen Nahinfrarot- und/oder Infrarotspektren und dem Gehalt an Oxidationsprodukt einer Aminosäure herstellt;
d) Aufnahme von Nahinfrarot- und/oder Infrarotspektren der Keratinfasern eines Individuums;
e) Bestimmung eines Schädigungsgrades der Keratinfasern dieses Individuums anhand des Kalibriermodells;
f) Ausgabe einer individuellen Behandlungsanweisung zu den Keratinfasern des Individuums in Abhängigkeit vom ermittelten Schädigungsgrad
wobei die Aminosäure ausgewählt ist aus der Gruppe bestehend aus Tryptophan, Methionin, Tyrosin, Histidin und Lysin.

2. Verfahren nach Anspruch 1, **dadurch gekennzeichnet, dass** das Kalibriermodell aus den Schritten a) bis c) als gespeicherte Information auf einem lokalen Datenträger oder in einer Cloud vorliegt.

3. Verfahren nach einem der Ansprüche 1 oder 2, **dadurch gekennzeichnet, dass** Schritt d) bei einem Friseur, an einem Verkaufspunkt ("point of sale", POS) von Haarbehandlungsmitteln oder im privaten Bereich durchgeführt wird.

4. Verfahren nach einem der Ansprüche 1 bis 3, **dadurch gekennzeichnet, dass** Schritt d) durch ein smartes Endgerät gesteuert wird, vorzugsweise über eine vorab installierte App, welche besonders bevorzugt über einen QR-Code, einen NFC-Chip, einen Barcode oder einen RFID-Chip direkt bei einem Friseur oder an einem Verkaufspunkt ("point of sale", POS) von Haarbehandlungsmitteln heruntergeladen werden kann.

5. Verfahren nach einem der Ansprüche 1 bis 4, **dadurch gekennzeichnet, dass** der Schädigungsgrad in "% geschädigt" oder in relativen Angaben ("sehr stark, stark, mittel, schwach und gar nicht") optisch, insbesondere mittels einer Anzeigevorrichtung, oder akustisch, insbesondere mittels einer Sprachansage, vorzugsweise durch ein smartes Endgerät, ausgegeben wird.

6. Verfahren nach einem der Ansprüche 1 bis 5, **dadurch gekennzeichnet, dass** das Kalibriermodell weitere Faktoren, insbesondere kategoriale Faktoren, beispielsweise Ethnizität des Individuums, Alter des Individuums und/oder Haarfarbe des Indivduums, berücksichtigt.

7. Verfahren nach einem der Ansprüche 1 bis 6, **dadurch gekennzeichnet, dass** die individuelle Behandlungsanweisung die Empfehlung von Blondiermitteln und/oder Haarfärbemitteln und/oder Haarpflegeprodukten und/oder Haarstylingmitteln umfasst.

8. Verfahren nach einem der Ansprüche 1 bis 7, **dadurch gekennzeichnet, dass** die individuelle Behandlungsanweisung die Empfehlung umfasst, für einen bestimmten Zeitraum von Blondierungen und/oder oxidativem Färben und/oder permanenten Verformungen und/oder Hitzebehandlung Abstand zu nehmen.

9. Verfahren nach einem der Ansprüche 1 bis 7, **dadurch gekennzeichnet, dass** die individuelle Behandlungsanweisung eine Dosierungsempfehlung für eine Blondierung und/oder eine oxidative Färbung und/oder die Vorhersage eines Resultats einer Blondierung und/oder oxidativen Färbung umfasst.

10. Verfahren nach einem der Ansprüche 1 bis 9, **dadurch gekennzeichnet, dass** die individuelle Behandlungsanweisung darin besteht, dem Individuum zum/vom Einsatz von Haarbehandlungsprodukten, die das Individuum anhand von QR-Codes, NFC-Chips, Barcodes oder RFID-Chips, identifiziert, zu- oder abzuraten.

11. Verfahren nach einem der Ansprüche 1 bis 10, **dadurch gekennzeichnet, dass** die individuelle Behandlungsanweisung darin besteht, dem Individuum zum Einsatz von Blondiermitteln und/oder Haarfärbemitteln und/oder Haarpflegeprodukten und/oder Haarstylingmitteln, die für das Individuum individuell hergestellt werden, zu zuraten und einen Bestellvorgang, vorzugsweise durch Aufrufen einer Internetseite eines Herstellers von individuellen Blondiermitteln und/oder individuellen Haarfärbemitteln und/oder individuellen Haarpflegeprodukten und/oder individuellen Haarstylingmitteln, einzuleiten.

12. Verfahren nach einem der Ansprüche 1 bis 11, **dadurch gekennzeichnet, dass** die individuelle Behandlungsanweisung darin besteht, die chemische Zusammensetzung eines Haarbehandlungsmittels, insbesondere eines Blondiermittels, eines Haarfärbemittels, eines Haarpflegeprodukts und/oder eines Haarstylingmittels zu ermitteln.

13. Verfahren nach einem der Ansprüche 1 bis 12, **dadurch gekennzeichnet, dass** zusätzlich zum Schädigungsgrad der Keratinfasern deren Kräuselung bestimmt wird und diese bei der individuellen Behandlungsanweisung berücksichtigt wird.

14. Verfahren nach einem der Ansprüche 1 bis 13, **dadurch gekennzeichnet, dass** zusätzlich zum Schädigungsgrad der Keratinfasern deren Dicke bestimmt wird und diese bei der individuellen Behandlungsanweisung berücksichtigt wird.

15. Verfahren nach einem der Ansprüche 1 bis 14, **dadurch gekennzeichnet, dass** zusätzlich zum Schädigungsgrad der Keratinfasern deren Grauanteil bestimmt wird und diese bei der individuellen Behandlungsanweisung berücksichtigt wird.

## Claims

1. A method for determining an individualized hair treatment, **characterized by** the steps of
a) determining, by means of a chromatographic or colorimetric method, a content of an oxidation product of an amino acid of a plurality of samples of keratin fibers that have been damaged to varying degrees;
b) recording near-infrared and/or infrared spectra of the samples of keratin fibers that have been damaged to varying degrees;
c) creating a calibration model which establishes a correlation between the near-infrared and/or infrared spectra and the content of the oxidation product of an amino acid;
d) recording near-infrared and/or infrared spectra of the keratin fibers of an individual;
e) determining a degree of damage to the keratin fibers of this individual using the calibration model;
f) outputting individual treatment advice relating to the keratin fibers of the individual, according to the determined degree of damage,
wherein the amino acid is selected from the group consisting of tryptophan, methionine, tyrosine, histidine and lysine.

2. The method according to claim 1, **characterized in that** the calibration model from steps a) to c) is provided as stored information on a local data carrier or in a cloud.

3. The method according to one of claims 1 or 2, **characterized in that** step d) is carried out at a hair salon, at a point of sale (POS) for hair treatment agents or privately.

4. The method according to one of claims 1 to 3, **characterized in that** step d) is controlled by a smart terminal, preferably by means of a previously installed app, which can be particularly preferably downloaded directly at a hair salon or at a point of sale (POS) for hair treatment agents via a QR code, an NFC chip, a bar code or an RFID chip.

5. The method according to one of claims 1 to 4, **characterized in that** the degree of damage is output visually, in particular by means of a display device, or acoustically, in particular by means of a voice announcement, preferably by a smart terminal, as "% damage" or in relative terms ("very highly, highly, moderately, slightly or not at all damaged").

6. The method according to one of claims 1 to 5, **characterized in that** the calibration model takes into account further factors, in particular categorical factors, for example ethnicity of the individual, age of the individual and/or hair color of the individual.

7. The method according to one of claims 1 to 6, **characterized in that** the individual treatment advice comprises the recommendation of bleaching agents and/or hair dyes and/or hair care products and/or hair styling agents.

8. The method according to one of claims 1 to 7, **characterized in that** the individual treatment advice comprises the recommendation to refrain from bleaching and/or oxidative dyeing and/or permanent shaping and/or thermal treatment for a certain period of time.

9. The method according to one of claims 1 to 7, **characterized in that** the individual treatment advice comprises a dosage recommendation for bleaching and/or oxidative dyeing and/or the prediction of a result of bleaching and/or oxidative dyeing.

10. The method according to one of claims 1 to 9, **characterized in that** the individual treatment advice consists in encouraging the individual to use hair treatment products that the individual has identified using QR codes, NFC chips, bar codes or RFID chips, or discouraging the individual from using said products.

11. The method according to one of claims 1 to 10, **characterized in that** the individual treatment advice consists in encouraging the individual to use bleaching agents and/or hair dyes and/or hair care products and/or hair styling agents that are manufactured individually for the individual and to initiate an ordering process, preferably by visiting a website of a manufacturer of individual bleaching agents and/or individual hair dyes and/or individual hair care products and/or individual hair styling agents.

12. The method according to one of claims 1 to 11, **characterized in that** the individual treatment advice consists in determining the chemical composition of a hair treatment agent, in particular a bleaching agent, a hair dye, a hair care product and/or a hair styling agent.

13. The method according to one of claims 1 to 12, **characterized in that**, in addition to the degree of damage to the keratin fibers, the curl thereof is determined and taken into consideration in the individual treatment advice.

14. The method according to one of claims 1 to 13, **characterized in that**, in addition to the degree of damage to the keratin fibers, the thickness thereof is determined and taken into consideration in the individual treatment advice.

15. The method according to one of claims 1 to 14, **characterized in that**, in addition to the degree of damage to the keratin fibers, the amount of gray therein is determined and taken into consideration in the individual treatment advice.

## Revendications

1. Procédé pour établir un traitement capillaire personnalisé, **caractérisé par** les étapes
a) de détermination d'une teneur en produit d'oxydation d'un acide aminé de plusieurs échantillons de fibres kératiniques endommagées différemment au moyen d'un procédé chromatographique ou colorimétrique ;
b) d'enregistrement des spectres infrarouges proches et/ou infrarouges des échantillons de fibres kératiniques endommagées différemment ;
c) de création d'un modèle d'étalonnage qui établit une corrélation entre les spectres infrarouges proches et/ou infrarouges et la teneur en produit d'oxydation d'un acide aminé ;
d) d'enregistrement des spectres infrarouges proches et/ou infrarouges des fibres kératiniques d'un individu ;
e) de détermination d'un degré de détérioration des fibres kératiniques de cet individu à l'aide du modèle d'étalonnage ;
f) de fourniture d'une indication individuelle de traitement pour les fibres kératiniques de l'individu en fonction du degré de détérioration déterminé, l'acide aminé étant choisi dans le groupe constitué par le tryptophane, la méthionine, la tyrosine, l'histidine et la lysine.

2. Procédé selon la revendication 1, **caractérisé en ce que** le modèle d'étalonnage aux étapes a) à c) se présente sous forme d'informations stockées sur un support de données local ou dans un nuage.

3. Procédé selon l'une des revendications 1 ou 2, **caractérisé en ce que** l'étape d) est réalisée chez un coiffeur, dans un point de vente (POS) d'agents de traitement capillaire ou dans un cadre privé.

4. Procédé selon l'une des revendications 1 à 3, **caractérisé en ce que** l'étape d) est commandée par un terminal intelligent, de préférence au moyen d'une application installée au préalable, laquelle peut être directement téléchargée de manière particulièrement préférée chez un coiffeur ou dans un point de vente (POS) de produits de traitement capillaire, à l'aide d'un code QR, d'une puce NFC, d'un code-barre ou d'une puce RFID.

5. Procédé selon l'une des revendications 1 à 4, **caractérisé en ce que** le degré de détérioration est indiqué en « % d'endommagement » ou visuellement par des indications relatives (« très fortement, fortement, moyennement, faiblement et pas du tout »), notamment au moyen d'un dispositif d'affichage, ou acoustiquement, notamment au moyen d'une annonce vocale, de préférence par un terminal intelligent.

6. Procédé selon l'une des revendications 1 à 5, **caractérisé en ce que** le modèle d'étalonnage prend en compte d'autres facteurs, notamment des facteurs de catégorie, par exemple l'ethnie de l'individu, l'âge de l'individu et/ou la couleur des cheveux de l'individu.

7. Procédé selon l'une des revendications 1 à 6, **caractérisé en ce que** l'indication individuelle de traitement comprend la recommandation d'agents décolorants et/ou de colorants capillaires et/ou de produits de soins capillaires et/ou d'agents pour la mise en forme des cheveux.

8. Procédé selon l'une des revendications 1 à 7, **caractérisé en ce que** l'indication individuelle de traitement comprend la recommandation d'éviter toute décoloration et/ou coloration par oxydation et/ou permanente et/ou tout traitement thermique pendant une certaine période.

9. Procédé selon l'une des revendications 1 à 7, **caractérisé en ce que** l'indication individuelle de traitement comprend une recommandation de dosage pour une décoloration et/ou une coloration par oxydation et/ou la prévision d'un résultat d'une décoloration et/ou d'une coloration par oxydation.

10. Procédé selon l'une des revendications 1 à 9, **caractérisé en ce que** l'indication individuelle de traitement consiste à recommander ou à déconseiller à l'individu l'utilisation de produits de traitement capillaire, que l'individu identifie à l'aide de codes QR, de puces NFC, de codes-barres ou de puces RFID.

11. Procédé selon l'une des revendications 1 à 10, **caractérisé en ce que** l'indication individuelle de traitement consiste à recommander à l'individu l'utilisation d'agents décolorants et/ou de colorants capillaires et/ou de produits de soins capillaires et/ou d'agents pour la mise en forme des cheveux, lesquels sont fabriqués de manière personnalisée pour l'individu, et à initier un processus de commande, de préférence en consultant le site Internet d'un fabricant d'agents décolorants personnalisés et/ou de colorants capillaires personnalisés et/ou de produits de soins capillaires personnalisés et/ou d'agents personnalisés pour la mise en forme des cheveux.

12. Procédé selon l'une des revendications 1 à 11, **caractérisé en ce que** l'indication individuelle de traitement consiste à déterminer la composition chimique d'un agent de traitement capillaire, notamment d'un agent décolorant, d'un colorant capillaire, d'un produit de soin capillaire et/ou d'un agent pour la mise en forme des cheveux.

13. Procédé selon l'une des revendications 1 à 12, **caractérisé en ce qu'**en plus du degré de détérioration des fibres kératiniques, leur frisure est déterminée et est prise en compte dans l'indication individuelle de traitement.

14. Procédé selon l'une des revendications 1 à 13, **caractérisé en ce qu'**en plus du degré de détérioration des fibres kératiniques, leur épaisseur est déterminée et est prise en compte dans l'indication individuelle de traitement.

15. Procédé selon l'une des revendications 1 à 14, **caractérisé en ce qu'**en plus du degré de détérioration des fibres kératiniques, leur proportion de gris est déterminée et est prise en compte dans l'indication individuelle de traitement.
